(19)

Europäisches
Patentamt
European
Patent Office
Office européen
des brevets

(11)　EP 2 836 807 B1

(12) **EUROPEAN PATENT SPECIFICATION**

(45) Date of publication and mention
of the grant of the patent:
**12.09.2018  Bulletin 2018/37**

(21) Application number: **13714329.3**

(22) Date of filing: **19.02.2013**

(51) Int Cl.:
**G01N 21/87** *(2006.01)*　　**G01N 33/38** *(2006.01)*

(86) International application number:
**PCT/IB2013/000228**

(87) International publication number:
**WO 2013/124722 (29.08.2013 Gazette 2013/35)**

(54) **CALIBRATION OF A MEASURING DEVICE FOR MEASURING MATERIAL PROPERTIES OF DIAMONDS**

KALIBRIERUNG VON EINEM MESSINSTRUMENT ZUR MESSUNG DES MATERIALEIGENSCHAFTEN VON DIAMANTEN

ÉTALONNAGE D'UN INSTRUMENT DE MESURE DES PROPRIÉTÉS DE DIAMANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.02.2012  GB 201203108**

(43) Date of publication of application:
**18.02.2015  Bulletin 2015/08**

(73) Proprietor: **De Beers Centenary AG
6000 Luzern 6 (CH)**

(72) Inventor: **SMITH, James, Gordon, Charters
Buckinghamshire HP13 5QL (GB)**

(74) Representative: **Glowinski, Maurice
De Beers Technologies
Belmont Road
Maidenhead, Berkshire SL6 6JW (GB)**

(56) References cited:
**EP-A2- 2 407 763　　　WO-A1-95/24621
US-A1- 2002 161 566　　US-A1- 2010 088 348**

**Description**

**Field of the invention.**

The present invention refers to methods for the calibration or cross calibration of measuring devices used for the measurement of material properties of individual objects. The method may be applied to calibrate just one machine or may be applied to calibrate a plurality of machines possibly dispersed around the world thus providing a global standard. While the method disclosed has general applicability it is most beneficial for measuring devices that measure and possibly sort objects at high throughputs. The physical properties to be measured may be any single material property, which may include any physical, optical, electrical or chemical property of an object that is observable such as its mass or even its temperature, but more commonly the measurement will involve a plurality of dimensions. For example the method may be applied to the measure of the colour of an object which may in general be considered to be a measurement in three dimensions. It is well known, for example, that the colour of a picture element or pixel of a digital image may be parameterised by specifying the red green and blue values, although other parameterisations may be used such as the well-known CIE L* a* b* where L* refers to the apparent lightness of the object on a scale of 0 (black) to 100 (white) and a* and b* are colour differences in approximately green - red and blue - yellow dimensions.

Generally the each dimension of the measurement may be made on an interval scale and may be characterised by a real number. Mathematically a measurement of a single parameter such as an object's mass may be described as determining a scalar property while a measurement in multiple dimensions produces a vector with two, three or more components. Certain physical properties of an object, for example the Moment of Inertia may be more properly described using tensors, but for the purposes of this discussion the components of the tensor can be considered to form a vector. Furthermore it is possible to combine a number of different measurements e.g. mass, volume and three components of colour into a vector with multiple components and even to treat the measurement of a scalar property as a one dimensional vector. So where the invention is described as being applicable to a vector measurement it will be understood that this is not a limitation.

**Background of the invention:**

The general problem of efficiently calibrating or cross calibrating instruments is not new and many methods are known in the art. These methods may be characterised in creating a number of standard samples or standard conditions, observing the uncalibrated readings produced by the instrument under each conditions and calculating and applying suitable scaling to the uncalibrated data so that they match known target values. Once the scalings are known they may be applied to other uncalibrated observations to yield calibrated observations.

So, for example if a weighing machine were to be calibrated according to the prior art the standard conditions might be to record the observed weights for an empty pan and a standard weight of for example 100 grams. A table of data such as that below would be produced

| Condition / Sample | Calibrated target value(y) | Observed value (x) on uncalibrated instrument |
|---|---|---|
| 1. Empty pan | 0.0g | 5.0g |
| 2. Standard weight | 100.0g | 85.0g |

In this example it is obvious that the uncalibrated instruments readings correspond approximately with the weight but with appreciable error.

It is straight forward to show by the method of simultaneous equations that a more accurate measurement may be obtained by applying a linear transformation to the uncalibrated data as follows.

$$\text{Calibrated weight in grams} = 1.25 \times \text{uncalibrated weight in grams} - 6.25$$

So in this case the calibration involves determining two numbers which may be characterised as a scaling factor (1.25) and an offset (-6.25). Of course in any commercial weighing machine this procedure would be automated and the displayed results would be adjusted automatically so that after calibration the displayed or recorded readings were correct.

It is convenient to describe the parameters of the calibration as forming a calibration matrix In this case it would have the form [1.25 -6.25]. It is also convenient to employ the conventions of homogeneous coordinates so that the constant term is treated as though it is formed as a product of the offset term and an observed value that is always unity.

Under this convention the calibration matrix may be written

[1.25 -6.25]

[0.00 1.00]

An equivalent result may be obtained graphically drawing a straight line graph of observed weight on the x axis and the target weight on the y axis.

Current methods require the production of a single table where for a set of known conditions or standard objects the target values and observed uncalibrated values can be set out in a single table. It is also well understood that the calibration of an instrument may be improved by including more test sample or conditions and hence extending the data table to include more pairs of target values and observed values. This data may be used to produce a calibration either again by plotting a graph a drawing the best fit line or perhaps using the well-known technique of linear regression, also known as the method of least squares to estimate the gain and offset terms and possibly to include higher order corrections.

Regardless of the detailed method used, known techniques require an association between the target value and observed value for calibration condition so that the data may be set out in a single table. This seemingly trivial requirement causes considerable problems in situations where it is not possible or thoroughly impractical to pass individually identified calibration objects through a system such as a rough diamond sorting machine.

In the sorting machine, rough diamonds are fed from a hopper into a measurement cell which produces an uncalibrated measurement of the property to be measured for example weight or colour. Typically the rough diamonds are fed through the machine at an appreciable rate for example 7.5 stones / second in order to deal with the large numbers of small diamonds produced by a diamond mine. The diamonds may be dispensed into one of a plurality of dispense bins the particular bin being determined by the result of the measurement.

If the current methods of calibration are applied to the machine for the weighing or sorting of rough diamonds a number of problems occur which can be eliminated or mitigated by the present invention.

Firstly it is not practical or at least impractically slow to feed individual diamonds of known weight or colour into the machine to produce the required single table of calibration data. Secondly, the uncalibrated measurements from the machine may be subject to substantial random measurement errors. For example in the measurement of colour using an imaging system the orientation of the stone which may be random introduces considerable variability into the measurement. One way of mitigating this random error for an individual stones is to have a plurality of measurement channels for example 9 so that each stone is presented to each channel in turn or simultaneously but in a different orientation. By combining the results from each channel perhaps using an average technique employing the mean or median result the magnitude of the random error in the colour assigned to that stone may be reduced resulting in a more accurate sorting machine.

However during the calibration process each standard stone will typically produce different results for each channel as a result of the random error (even if all the channels were in perfect agreement.) Using current methodology this problem could be overcome by making repeated observations of the same test object, or using a batch of standard identical test objects. However in the case of the sorting machine neither of these options are available. Typically diamonds become dirty as they pass through a machine which would degrade their apparent colour and also high speed sorting machines are not designed to recycle individual stones. It will also be understood that no two rough diamonds are the same so it is not possible to assemble a batch of identical diamonds. It is possible to use a batch of other nominally identical objects such as coloured balls but it has been found that because of their unusual optical properties a sorting machine cannot be calibrated reliably using non - diamond tests objects. US 2009/0182520 A1 discloses a method for measuring the colour of diamonds.

The present invention aims to solve the problems associated with the current methodology of cross calibrating measuring devices. The following discussion describes some possible embodiments of the invention. It is not intended to be limiting and it will be appreciated that variations from the specific examples below may still fall within the scope of the invention.

**Detailed description:**

Accordingly the present invention in accordance with the independent clam 1 provides a novel method of cross calibrating measuring devices that allows calibrations to be carried out without a direct association between individual target values and their observed values. By breaking this link calibration samples used to cross calibrate two or more measuring devices may be used without regard to the order in which the individual diamonds in the sample are measured and so allows a calibration to be carried out in a greatly reduced time. By working the method of the invention it also becomes practical to carry out a calibration using for example many thousands of calibration diamonds thus reducing the effect of random errors of observation on the calibration, without creating an unmanageable number of individually identified samples.

In a first embodiment of the invention a measuring device may be calibrated or two or more measuring devices may be cross calibrated by providing a sample of three or more diamonds to be measured to which target statistics for one or more material properties has been assigned wherein said target statistics are independent of the order of presentation of individual diamonds; Providing observed statistics for the sample of three or more of said diamonds wherein the observed data set is recorded from the measuring device and wherein said observed statistics are independent of the order of presentation of individual diamonds and transforming the actual measurement output of the measuring device so that the transformed observed statistics match the target statistics. The transformation can also be applied to uncalibrated measurements of the measuring device to provide calibrated measurements.

In a further embodiment of the present invention the method is used to maintain the calibration of two or more measuring devices.

According to the present invention the target statistics are generated by providing a sample of three or more diamonds to be measured; measuring one or more material properties for sufficient diamonds in the sample in a first measuring device without regard to order; recording a target data set comprising the said measurements; and calculating target statistics from the target data set.

It will be understood that the target statistics for a particular sample may be obtained by any convenient method. For a first sample these may even be assigned arbitrarily.

One convenient method of obtaining the target statistics for a sample may be to pass the sample through a first machine (which then might be considered to be a master machine) and obtain a first set of observed statistics, to which the label of target statistics for that sample are assigned.

Similarly one might obtain a plurality of observed statistics for that sample from a plurality machines that are considered trustworthy, or by passing the sample through a master machine several times, pool the observed statistics in accordance to the standard mathematical procedures for combining data sets, and assigning these pooled observed statistics as the target statistics for the said sample.

In a further embodiment of the present invention the observed statistics are generated by providing the same sample or an essentially identical sample of three or more diamonds to be measured, measuring one or more material properties for sufficient diamonds in the sample in one or more measuring devices without regard to order of presentation of individual diamonds, recording an observed data set comprising all the measurements of said one or more material properties for the sample and assigning observed statistics to the observed data set.

It will be understood that the target statistics for a particular sample may be obtained by any convenient method. For a first sample these may even be assigned arbitrarily.

One convenient method of obtaining the target statistics for a sample may be to pass the sample through a first machine (which then might be considered to be a master machine) and obtain a first set of observed statistics, to which the label of target statistics for that sample are assigned.

Similarly one might obtain a plurality of observed statistics for that sample from a plurality machines that are considered trustworthy, or by passing the sample through a master machine several times, pool the observed statistics in accordance to the standard mathematical procedures for combining data sets, and assigning these pooled observed statistics as the target statistics for the said sample.

The method is applicable to calibration with a low number of individual diamonds in the range 3 to 100, such as 3 or 5 or 10 or 20 or 50 or 100 individual diamonds. The method is also applicable to larger numbers of individual diamonds in the range of 100 to 1,000 such as 200 or 500 or 750 individual diamonds. Furthermore the method is applicable to individual diamonds in the range 1,000 to 10,000 such as 2,000 or 5,000 or 7,500 individual diamonds. There may also be situations where even larger numbers of individual diamonds may be used to calibrate measuring equipment in the range 10,000 to 100,000 individual diamonds such as 20,000 or 50,000 or 75,000 successfully using this method. It is envisaged that in some technical fields the number of individual diamonds used to calibrate the measuring device would exceed 500,000 or even 1,000,000 individual diamonds.

The method of present invention will now be described by two examples.

**Calibration of a single parameter - a high speed weighing machine**

It is assumed that the weighing machine produces by whatever means an uncalibrated weight that is subject to a systematic measurement error which is to be reduced or eliminated by calibration. In addition each observation is subject to a random measurement error which may not be negligible. There is only one measurement result per stone observed.

A mathematical transformation of the uncalibrated data is defined. For this example the transformation will be assumed to be a linear transformation of the form discussed earlier so that the calibration process will involve the determination of a gain and an offset.

In general the transformation to be used will take into account the variations that the designer of the instrument might expect to occur or may be determined empirically based on the behaviour of for example a number of different prototypes. It is convenient if the transformation is a linear transformation but this is not essential.

If the final result of the measurement is produced as a result of a series of calculations it may be more appropriate to carry out the calibration using the intermediate values rather than the final result. This is especially the case if the final measurement involves a non-linear transformation of the intermediate results.

It is desirable for the transformation to be as simple as possible to achieve the desired effect with the minimum number of parameters. Including extra parameters may result in over fitting which delivers better performance to training data but does not give poorer results when applied to other situations. For example - in the case of the weighing machine if it is known a priori that a (hypothetical) object of zero mass will give uncalibrated readings with a mean of zero then it might be desirable to omit the offset term in the transformation.

A parcel of diamonds for example containing for example 1000 or 2000 diamonds is assembled. The weights of each stone are recorded by any convenient means. This could be by weighing each stone individually on a conventional balance or passing the stones through a high speed weighing machine that has already been calibrated. This parcel may be known as Calibration parcel. It is advisable for the goods in the Calibration parcel to have a distribution of weights that in some sense matches the distribution of weights the machine is likely to see in normal production so that the calibration has taken into account the full range and distribution of observations likely to be encountered.

Optionally a further parcel may be prepared known as a Verification parcel. This is kept to test that the calibration process has been successful and may be employed at later times to ensure that the machine remains in calibration.

Verification of the calibration of the machine may be carried out by

(i) Providing a verification sample of three or more diamonds to be measured to which verification target statistics for one or more material properties has been assigned wherein said verification target statistics are independent of the order of presentation of individual diamonds;

(ii) providing observed verification statistics for the sample of three or more of said diamonds wherein the observed verification data set is recorded from the measuring device and wherein said observed verification statistics are independent of the order of presentation of individual diamonds and;

(iii) Comparing the observed verification statistics of the measuring device with the target verification statistics and testing that they match within a pre-determined tolerance.

For example in a weighing machine it may be that a tolerance of the mean weights of 1% or 0.1% would be acceptable. In some contexts it might make sense to specify a tolerance in absolute units for example one gram or one milligram rather than a percentage. It will be understood that the skilled artisan would understand that the specific tolerances may vary from application to application.

A table of data of target weights for the parcels is assembled most conveniently in a computer data file and possibly stored on a database. The data need not be listed in any particular order although there is no harm in sorting the data perhaps into ascending order to facilitate the calculation of histograms or cumulative curves.

From this data at least one but preferably a plurality of statistical properties are calculated which may be known as Target Statistics. For the purpose of this example the mean and the standard deviation may serve as Target Statistics but other properties may be used such as the histogram including the median (50th percentile) and the interquartile range. The choice of Target Statistics to use will be based on the particular circumstances of the measurement and it is not possible to prescribe the optimal choice in advance but those described here will be sufficient to work the invention to a reasonable degree of effectiveness. Optionally further Target Statistics may be defined such as the cumulative curve of weights perhaps measured at every centile. These further Target Statistics need not be used in calculating the parameters of the Transformation but may be used to confirm that the distribution produced by calibration has similar statistical properties.

The Calibration Parcel is fed through the machine to be calibrated producing a second table of data which may be known as the Observed Data. The Observed Data is treated in the same way as for the Calibration Date to produce a set of Observed Statistics. These will in general be close to but not exactly matching the Target Statistics.

It will be noted that if both the Calibrated and Observed data sets are sorted into for example ascending order and tabulated there might be a reasonable chance that the paired data may come from the same object in significant portion of cases, especially if the number of data points is small and the random measurement errors are insignificant. While the invention does not exclude making use of this association it is not an essential feature.

The parameters of the transformation are now determined. This may be carried out by any convenient method. For the example set out here involving a linear transformation the gain term may be determined as the ratio of the Target Standard deviation to the Observed standard deviation. It is then straight forward to calculate the new transformed mean assuming no offset. The calibration offset may then be calculated as the difference between the Target and transformed Observed means.

Other methods may be used to determine the transformation. It would be within the scope of the invention to determine the transformation by iterative means or "by eye". For example a computer spreadsheet program such as Microsoft Excel TM may be programmed to display for example the cumulative plots of the Target and transformed Observed data, the transformation being determined by cells on a sheet. These cells may be adjusted manually until the curves overlap to the satisfaction of the operator. By defining a measure of deviation - such as the Kolmogorov-Smirnov distance the Excel Solver may be used to minimise the observed deviation between the two curves.

Once the transformation is determined it is desirable to compare the Further Target Values with those calculated for the transformed Observed values to determine if the Transformation does give a good match.

Once the coefficients of the transformation have been determined they may be used in any convenient means. Usually this will involve passing the calibration coefficients to the instrument so that further results are automatically scaled by the transformation. For example they may be saved to a computer file in the form of a calibration matrix in homogeneous coordinates as described earlier.

The invention may also be applied to correct earlier recorded but uncalibrated observations.

## Calibration of three parameters - a high speed colour measurement machine

In the colour measurement machine means are provided to transfer individual diamonds from an input hopper via a measurement cell to an output possibly with a plurality of dispense bins. A plurality of colour measurement channels are provided in the measurement cell each arranged so that the diamond is observed from a different orientation so that the apparent colour of the object from that orientation is determined. Each channel provides a measurement of colour in the form of three numbers which may range without limitation from 0.0 to 1.0, the three numbers representing the red, green and blue values which may be considered to form a row vector $\{r, g, b, 1.0\}$ where the 1.0 represents the constant term following the convention of homogeneous co-ordinates.

It will be understood that a white or colourless object might be expected to have values approaching 1.0 for r, g and b and a black object values close to 0.0. A yellow object, a common colour in diamonds will have a b value that is less than that for the green channel while a brown object another common colour in diamonds will tend to have both g and b values less than that recorded for the r channel.

The calibration of this instrument involves finding for each channel a calibration matrix that transforms the data produced by that channel into a standard result. In general it is not convenient to present the results of colour measurement of a diamond in terms of r g and b values. Instead they are first transformed by a linear transformation into 3 other dimensions that express the results in a more useful form. Many options are available for this transformation, including conversion to the well-known CIE X, Y Z system. However for the purposes of illustrating the invention a simpler but satisfactory transformation may be obtained by making use the following transformation using colour differences.

$$y = g$$

$$a = r\text{-}g$$

$$b = g\text{-}b$$

It will be understood that this transformation may be performed by a matrix multiplication on the original data vector.

In this transformed space, a and b would ideally be 0.0 for both black and white (or colourless) objects, the overall lightness level being encoded in the y channel. Yellow objects would tend to have positive b values and approximately zero for a. Brown objects would tend to have positive a and b values.

One method of calibrating the colour machine according to this invention is to apply the method of the first example to the three signals y a and b independently. Observed Statistics for y a and b are obtained from each channel of the machine for Calibration Parcel and a transformation for each feature and channel is found to match pre-defined Target Statistics.

It is advantageous if the Calibration Parcel contains a full range of colours that the machine is likely to encounter in production so that calibration is balanced over this range and does not fail when it encounters diamond colours that have not been encountered during calibration.

In practice applying the method of the first example to the calibration of a colour measurement machine is not entirely satisfactory. While the results for each channel for y are well aligned there were systematic errors in the results for a and b. In particular a scatter plot of for example b vs. y for the channels showed that the calibrated results while correct on average showed an error or trend that was correlated with y. This trend varied from one channel to the next and was presumably associated with subtle differences in the apparatus for each channel.

Thus in a second embodiment of the invention instead of treating the three channels y a and b independently it is advantageous to take account of correlations between the Observed Values of these channels and match them to correlations seen in the target values.

For example as discussed above it was found that an adequate calibration of the y signal could be obtained using the method previously set out but the a and b signals required an adjustment to compensate out the correlation with the y signal. This may be carried out by a transformation that for the a and b channels mixes in a small proportion of the y channel into the a and b channel signals.

Thus for b the calibration transformation becomes

$$b' = gain * b + e * y + offset$$

Where the term e is a cross or off diagonal term in full calibration matrix, while the gain and offset are recognisable from the previous example (but may not be numerically the same).

While any convenient method may be used to find the gain, offset and off diagonal term the following procedure was found to be effective.

A calibration parcel of for example 4000 diamonds of different colours and is passed through the machine and the Observed Values of y a and b are recorded for each channel. A check is made that sufficient number of the stones have been observed - for example 95% so that the observed data is reasonably representative of the entire parcel.

The means of y a and b are recorded as Observed Statistics. The Variances and Covariances of the data are computed. This produces a 3x3 symmetric matrix, known as a Covariance Matrix with the Variances on the diagonal and the Covariances off the diagonal.

From the Covariance Matrix it is straightforward to calculate the Correlation Coefficient of for example y and b. The Correlation coefficient is a number between -1 and 1 that describes the degree of linear correlation between the two variables. It had the advantage that it is not altered by scaling of either variable. However it can be influenced by mixing for example some of the y signal into the b signal.

Thus the calibration proceeds by first finding how much of the y signal needs to be mixed into the b signal so that the correlation coefficient of the transformed b signal matches that for the Target statistics. An error may be defined as = (Observed correlation coefficient - target correlation coefficient).

An approximate value for the correction term e may be found as using the formula

$$e = -error. \ sqrt(Var(b)/Var(y)).$$

Substitution of this value into transformation will give an improved version of b with a reduced error in correlation coefficient. It is then straightforward to use iteration to find further estimates of e that will rapidly converge to a value that yields the target correlation coefficient.

Once the correlation has been corrected further scaling and offset transformations of the revised transformed b or y values will not disturb the correlation coefficient. So it will be seen that the calibration of y and the transformed b values may proceed directly using the method already described in the first example.

This will provide a second transformation to be applied after the first. Using the well-known results of matrix algebra it is then possible to combine these two transformations into one calibration matrix by matrix multiplication.

## Claims

1. A method of calibrating a measuring device for measuring material properties of diamonds comprising:

    (i) Providing a sample of three or more diamonds to be measured to which target statistics for one or more material properties has been assigned wherein said target statistics are independent of the order of presentation of individual diamonds;
    (ii) producing observed statistics for the sample based on the observed data set of the sample recorded from the measuring device and wherein said observed statistics are independent of the order of presentation of individual diamonds and;
    (iii) transforming the actual measurement output of the measuring device so that the transformed observed statistics match the target statistics.

    Wherein the target statistics are generated by:

(i) providing a sample of three or more diamonds to be measured;
(ii) measuring one or more material properties for sufficient diamonds in the sample in a first measuring device without regard to order of presentation of individual diamonds and;
(iii) recording a target data set comprising the said measurements; and calculating target statistics from the target data set.

2. The method of claim 1 further comprising applying the transformation to uncalibrated measurements of a measuring device for measuring material properties of diamonds to provide calibrated measurements.

3. The method of any of the previous claims wherein the target statistics are generated by:

(i) providing the same sample or an essentially identical sample of three or more diamonds to be measured;
(ii) measuring one or more material properties for sufficient diamonds in the sample in one or more measuring devices without regard to order of presentation of individual diamonds;
(iii) recording an target data set comprising all the measurements of said one or more material properties for the sample and;
(iv) assigning target statistics to the observed data set.

4. The method of any of the previous claims for maintaining the calibration of two or more measuring devices for measuring material properties of diamonds.

5. The method of any of the previous claims further comprising the step of verifying the calibration of the measuring device for measuring material properties of diamonds comprising:

(i) providing a verification sample of three or more diamonds to be measured to which verification target statistics for one or more material properties has been assigned wherein said verification target statistics are independent of the order of presentation of individual diamonds;
(ii) producing observed verification statistics for the sample of three or more of said diamonds based on the observed verification data set of the sample recorded from the measuring device and wherein said observed verification statistics are independent of the order of presentation of individual diamonds and;
(iii) comparing the observed verification statistics of the measuring device with the target verification statistics and testing that they match within a pre-determined tolerance.

6. The method of any of the previous claims wherein the diamonds are rough diamonds.

7. The method of any of the previous claims wherein the measured properties of the diamonds are colour, mass, volume and moment of inertia.

**Patentansprüche**

1. Verfahren zur Kalibrierung eines Messinstruments zur Messung der Materialeigenschaften von Diamanten, umfassend:

(i) Bereitstellen einer Probe von drei oder mehr zu messenden Diamanten, denen eine Sollstatistik für eine oder mehrere Materialeigenschaften zugeordnet wurde, wobei die Sollstatistik von der Reihenfolge der Präsentation der einzelnen Diamanten unabhängig ist;
(ii) Erstellen der beobachteten Statistik für die Probe auf der Grundlage des beobachteten Datensatzes der Probe, der vom Messinstrument aufgezeichnet wurde, und wobei die beobachtete Statistik von der Reihenfolge der Präsentation der einzelnen Diamanten unabhängig ist und;
(iii) Umwandeln des Ist-Messausgangs des Messinstruments, so dass die umgewandelte beobachtete Statistik der Sollstatistik entspricht.

Wobei die Sollstatistik erzeugt wird durch:

(i) Bereitstellen einer Probe von drei oder mehr zu messenden Diamanten;
(ii) Messen einer oder mehrerer Materialeigenschaften für ausreichend Diamanten in der Probe in einem ersten Messinstrument ungeachtet der Reihenfolge der Präsentation der einzelnen Diamanten und;

(iii) Aufzeichnen eines Zieldatensatzes umfassend die Messungen; und Berechnen der statistischen Zieldaten aus dem Zieldatensatz.

**2.** Verfahren nach Anspruch 1, ferner umfassend Anwenden der Umwandlung auf die unkalibrierten Messungen eines Messinstruments zur Messung von Materialeigenschaften von Diamanten, um kalibrierte Messungen bereitzustellen.

**3.** Verfahren nach einem der vorhergehenden Ansprüche, wobei die Zielstatistik erzeugt wird durch:

(i) Bereitstellen derselben Probe oder einer im Wesentlichen identischen Probe von drei oder mehr zu messenden Diamanten;
(ii) Messen einer oder mehrerer Materialeigenschaften für ausreichend Diamanten in der Probe in einem oder mehr Messinstrumenten ungeachtet der Reihenfolge der Präsentation der einzelnen Diamanten;
(iii) Aufzeichnen eines Zieldatensatzes, umfassend alle Messungen der einen oder mehreren Materialeigenschaften für die Probe und;
(iv) Zuordnen der Zielstatistik zu dem beobachteten Datensatz.

**4.** Verfahren nach einem der vorhergehenden Ansprüche zur Aufrechterhaltung der Kalibrierung von zwei oder mehr Messinstrumenten zur Messung der Materialeigenschaften von Diamanten.

**5.** Verfahren nach einem der vorhergehenden Ansprüche, ferner umfassend den Schritt des Überprüfens der Kalibrierung des Messinstruments zur Messung der Materialeigenschaften von Diamanten, umfassend.

(i) Bereitstellen einer Überprüfungsprobe von drei oder mehr zu messenden Diamanten, denen eine Überprüfungssollstatistik für eine oder mehrere Materialeigenschaften zugeordnet wurde, wobei die Überprüfungssollstatistik von der Reihenfolge der Präsentation einzelner Diamanten unabhängig ist;
(ii) Bereitstellen der beobachteten Überprüfungsstatistik für die Probe von drei oder mehr der Diamanten auf der Grundlage des beobachteten Datensatzes der Probe, der vom Messinstrument aufgezeichnet wurde, und wobei die beobachtete Überprüfungsstatistik von der Reihenfolge der Präsentation der einzelnen Diamanten unabhängig ist und;
(iii) Vergleichen der beobachteten Überprüfungsstatistik des Messinstruments mit der Überprüfungssollstatistik und Testen, ob sie innerhalb einer vorbestimmten Toleranz übereinstimmen.

**6.** Verfahren nach einem der vorhergehenden Ansprüche, wobei die Diamanten Rohdiamanten sind.

**7.** Verfahren nach einem der vorhergehenden Ansprüche, wobei die gemessenen Eigenschaften der Diamanten Farbe, Masse, Volumen und Trägheitsmoment sind.

**Revendications**

**1.** Procédé d'étalonnage d'un dispositif de mesure pour la mesure de propriétés matérielles de diamants, comprenant :

(i) la mise à disposition d'un échantillon de trois diamants ou plus à mesurer auxquels des statistiques cibles pour une ou plusieurs propriétés matérielles ont été attribuées, lesdites statistiques cibles étant indépendantes de l'ordre de présentation de diamants individuels ;
(ii) la production de statistiques observées pour l'échantillon à partir de l'ensemble de données observé de l'échantillon enregistré à partir du dispositif de mesure, lesdites statistiques observées étant indépendantes de l'ordre de présentation de diamants individuels, et
(iii) la transformation de la sortie de mesure réelle du dispositif de mesure de telle sorte que les statistiques observées transformées correspondent aux statistiques cibles,

les statistiques cibles étant générées par :

(i) la mise à disposition d'un échantillon de trois diamants ou plus à mesurer ;
(ii) la mesure d'une ou de plusieurs propriétés matérielles pour des diamants suffisants dans l'échantillon dans un premier dispositif de mesure sans prendre en compte l'ordre de présentation de diamants individuels, et
(iii) l'enregistrement d'un ensemble de données cible comprenant lesdites mesures ; et le calcul de statistiques cibles à partir de l'ensemble de données cible.

**2.** Procédé selon la revendication 1, comprenant en outre l'application de la transformation à des mesures non étalonnées d'un dispositif de mesure pour la mesure de propriétés matérielles de diamants pour obtenir des mesures étalonnées.

**3.** Procédé selon l'une quelconque des revendications précédentes, dans lequel les statistiques cibles sont générées par :

(i) la mise à disposition du même échantillon ou d'un échantillon essentiellement identique de trois diamants ou plus à mesurer ;
(ii) la mesure d'une ou de plusieurs propriétés matérielles pour des diamants suffisants dans l'échantillon dans un ou plusieurs dispositifs de mesure sans prendre en compte l'ordre de présentation de diamants individuels ;
(iii) l'enregistrement d'un ensemble de données cible comprenant toutes les mesures de ladite ou desdites propriétés matérielles pour l'échantillon, et
(iv) l'attribution de statistiques cibles à l'ensemble de données observé.

**4.** Procédé selon l'une quelconque des revendications précédentes pour maintenir l'étalonnage de deux dispositifs de mesure ou plus pour la mesure de propriétés matérielles de diamants.

**5.** Procédé selon l'une quelconque des revendications précédentes, comprenant en outre l'étape de vérification de l'étalonnage du dispositif de mesure ou plus pour la mesure de propriétés matérielles de diamants comprenant :

(i) la mise à disposition d'un échantillon de vérification de trois diamants ou plus à mesure auxquels des statistiques cibles de vérification pour une ou plusieurs propriétés matérielles ont été attribuées, lesdites statistiques cibles de vérification étant indépendantes de l'ordre de présentation de diamants individuels ;
(ii) la mise à disposition de statistiques de vérification observées pour l'échantillon de trois ou plus desdits diamants à partir de l'ensemble de données de vérification observé de l'échantillon enregistré à partir du dispositif de mesure, lesdites statistiques de vérification observées étant indépendantes de l'ordre de présentation de diamants individuels, et
(iii) la comparaison des statistiques de vérification observées du dispositif de mesure avec les statistiques de vérification cibles et la vérification de leur correspondance avec une tolérance prédéterminée.

**6.** Procédé selon l'une quelconque des revendications précédentes, dans lequel les diamants sont des diamants bruts.

**7.** Procédé selon l'une quelconque des revendications précédentes, dans lequel les propriétés mesurées des diamants sont la couleur, la masse, le volume et le moment d'inertie.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20090182520 A1